# EUROPEAN PATENT APPLICATION

(11) **EP 1 396 251 A1**
(43) Date of publication of application: **10.03.2004**
(21) Application number: 02722828.7
(22) Date of filing: 26.04.2002
(51) Int. Cl.: A61K 6/00, A61K 7/18, A61C 1/08, A61P 1/02, C09D 5/00, C09D 1/00, C09D 7/12

(54) **FLUORINE COATING COMPOSITION AND METHOD OF FLUORINE COATING**

(30) Priority: 27.04.2001 JP 2001132804
(71) Applicant: Sugihara, Shinichi, Yokohama-shi, Kanagawa 227-0052 (JP)
(72) Inventor: Sugihara, Shinichi, Yokohama-shi, Kanagawa 227-0052 (JP)
(74) Representative: Gervasi, Gemma, Dr.
(86) International application number: PCT/JP2002/004219
(87) International publication number: WO 2002/087514

(57) **Abstract**

A composition for dental fluorine coating comprising a fluorine-containing compound and a photocatalyst. A dental fluorine coating method comprising the steps of applying the above dental fluorine coating composition to the surface of a tooth and irradiating the resultant coating with light. A desired fluorine coating effect is obtainable more rapidly than prior art.

## Description

### Technical Field

The present invention relates to a composition for dental fluorine coating comprising a fluorine containing compound and a photocatalyst, and a dental fluorine coating method.

### Background Art

Dental fluorine coatings are widely employed to prevent dental caries. However, dental fluorine coatings must be applied at least a few times at regular intervals.

Accordingly, the object of the present invention is to provide a composition for dental fluorine coating, and fluorine coating method, achieving a substantial fluorine coating effect with a single dental fluorine coating.

### Disclosure of the Invention

The present invention relates to a dental fluorine coating composition comprising a fluorine-containing compound and a photocatalyst.

In the fluorine coating composition of the present invention, the photocatalyst may be UV-responsive titanium oxide or visible light responsive titanium oxide. From the perspective of permitting the use of light of long wavelength, the photocatalyst (referred to hereinafter as a "visible light responsive photocatalyst") is one that is activated by the action of light with a wavelength of greater than or equal to 420 nm, desirably one that is activated by the action of light with a wavelength of greater than or equal to 450 nm, in the form of visible light responsive titanium oxide. The visible light responsive type photocatalyst may be a visible light responsive type material comprising at least anatase type titanium dioxide, as well as the main signal having a g value ranging from 2.004 to 2.007 and two subsignals having a g value ranging from 1.985 to 1.986 and a g value at 2.024 are observed in the ESR measured under the irradiation of light having a wavelength of 420 nm or more at 77 K in vacuum, and said three signals are observed in a little amount or substantially not observed in darkness at 77 K in vacuum. The visible light responsive type photocatalyst may be various visible light responsive type materials mentioned below.

The present invention further relates to a dental fluorine coating method comprising the steps of applying the above-described composition of the present invention to the surface of a tooth and irradiating the coating with light, and a dental fluorine coating method comprising the steps of applying the above-described composition of the present invention to the surface of a tooth and irradiating the coating with light comprising a component with a wavelength of greater than or equal to 420 nm.

In the fluorine coating methods of the present invention, the light is desirably emitted by a photopolymerizer, light-emitting diode, or halogen lamp, or is plasma light. The light-emitting diode is desirably a violet-emitting diode, blue-emitting diode, green-emitting diode, yellow-emitting diode, or white-emitting diode.

### Brief Description of the Drawings

Fig. 1 shows ESR spectra of the visible light responsive type material of the present invention (Manufacturing Example 1) measured at 77 K in vacuum. The upper segment is a spectrum measured in darkness, the middle segment is a spectrum measured with irradiation by light (light below 420 nm was cut off in light from a mercury lamp) having a wavelength of not less than 420 nm, and the lower segment is a spectrum measured with irradiation with light from a mercury lamp from which light of less than 420 nm was not cut off.
Fig. 2 shows ESR spectra of the visible light responsive type material of the present invention (Manufacturing Example 1) measured at ordinary temperature in vacuum. The upper segment is a spectrum measured in darkness, the middle segment is a spectrum measured with irradiation by light (light below 420 nm was cut off in light from a mercury lamp) having a wavelength of not less than 420 nm, and the lower segment is a spectrum measured with irradiation with light from a mercury lamp from which light of less than 420 nm was not cut off.
Fig. 3 gives the results of measurement by XRD of the product of Manufacturing Example 1 (upper segment) and a hydrolysis product (dried at 50°C) (lower segment).
Fig. 4 shows light-emitting spectra of a blue light-emitting diode (NSPB), green light-emitting diode (NSPG) and white light-emitting diode (NSPW).
Fig. 5 shows ESR spectra of Manufacturing Example 4 (the visible light responsive type material) measured at 77 K in vacuum with irradiation by light (light below 420 nm was cut off in light from a mercury lamp) having a wavelength of not less than 420 nm.
Fig. 6 shows ESR spectra of Manufacturing Example 5 (the visible light responsive type material) measured at 77 K in vacuum with irradiation by light (light below 420 nm was cut off in light from a mercury lamp) having a wavelength of not less than 420 nm.
Fig. 7 shows ESR spectra of Manufacturing Example 6 (the visible light responsive type material) measured at 77 K in vacuum with irradiation by light (light below 420 nm was cut off in light from a mercury lamp) having a wavelength of not less than 420 nm.

### Best Mode of Implementing the Invention

### [Fluorine Coating Composition]

The dental fluorine coating composition of the present invention comprises a fluorine-containing compound and a photocatalyst. The fluorine-containing compound may be in the form of one or more of the compounds conventionally employed in dental fluorine coatings. Examples are: sodium fluoride and sodium phosphorus fluoride. Stannous fluoride, acidic fluorophosphoric acid solutions, and silver ammine fluoride may also be suitably employed. The photocatalyst may be UV-responsive titanium oxide or visible light-responsive titanium oxide. For example, UV-responsive titanium oxide may be in the form of commercial ST-01 (made by Ishihara Sangyo K.K.) or P25 (made by Nippon Aerosil).

The contents of the fluorine-containing compound and photocatalyst may be set appropriately, for example, the content of fluorine-containing compound is from 0.1 to 90 weight percent, desirably from 1 to 30 weight percent, and the content of the photocatalyst is from 0.1 to 30 weight percent, preferably from 1 to 10 weight percent.

The fluorine coating composition of the present invention may be in the form of a solution, gel, or the like, and may comprise, in addition to the above-described fluorine-containing compound and photocatalyst, a gelling agent, buffer, water, solvent, fragrance materials, stabilizers, and other suitable known components.

The visible light responsive type photocatalyst used for the fluorine coating composition of the present invention may be, for example, a visible light responsive type material in the form of titanium oxide comprising at least anatase titanium oxide, with a main signal (the strongest signal) having a g value ranging from 2.004 to 2.007 and two subsignals (signals of lesser intensity than the main signal) having a g value ranging from 1.985 to 1.986 and a g value at 2.024 as measured by ESR under the irradiation of light having a wavelength of 420 nm or more at 77 K in vacuum. With the visible light responsive type material, the three signals (one main signal and two subsignals) are observed in a little amount or substantially not observed in darkness at 77 K in vacuum.

The light having a wavelength of not less than 420 nm employed in ESR is obtained by passing light from a high-pressure mercury lamp (for example, 500 W) through a filter (L-42) cutting out light with a wavelength below 420 nm.

Further, even in ESR measurement conducted with irradiation by light having a wavelength of not less than 455 nm at 77 K in vacuum (obtained by passing light from a xenon lamp (for example, 150 W) through a filter (GG455) cutting light of wavelengths below 455 nm), the visible light responsive type material sometimes exhibits a main signal (the strongest signal) having a g value ranging from 2.004 to 2.007 and two subsignals (signals of lesser intensity than the main signal) having a g value ranging from 1.985 to 1.986 and a g value at 2.024.

The visible light responsive type material used for the coating composition of the present invention may be, for example, a visible light photocatalyst comprised of titanium dioxide having stable oxygen defects described in WO 00/10706. The ESR spectrum of the visible light photocatalyst described in WO 00/10706 has only a signal having a g value ranging from 2.003 to 2.004 in ESR measurement in the dark at 77 K in vacuum. The visible light photocatalyst described in WO 00/10706 is heterogenic with the spectrum exhibited by the above-mentioned visible light responsive type material. However, both exhibit activity with visible light having a wavelength of not less than 420 nm, preferably visible light having a wavelength of not less than 450 nm.

Among the visible light responsive type materials, typical ones will be set forth below.

The visible light responsive type material used in the present invention is preferably titanium oxide comprising a main component in the form of anatase titanium oxide, and may additionally comprise amorphous titanium oxide. Alternatively, it may comprise rutile titanium oxide, and further comprise amorphous titanium oxide other than rutile titanium oxide. Further, the anatase titanium oxide and rutile titanium oxide need not necessarily have a high degree of crystallinity.

Still further, the titanium oxide constituting the visible light responsive type material may comprise titanium and oxygen in non-stoichiometric ratio. Specifically, the quantity of oxygen relative to titanium may be lower than the stoichiometric ratio (a theoretic value of 2.00) of titanium dioxide. In the titanium oxide in the visible light responsive type material, the molar ratio of oxygen to titanium may be, for example, less than 2.00, for example, 1.00-1.99, or 1.50-1.99. The molar ratio of oxygen to titanium in the titanium oxide in the visible light responsive type material can be measured, for example, by X-ray photoelectron spectroscopy. The visible light responsive type material may or may not comprise nitrogen or nitrogen derivatives.

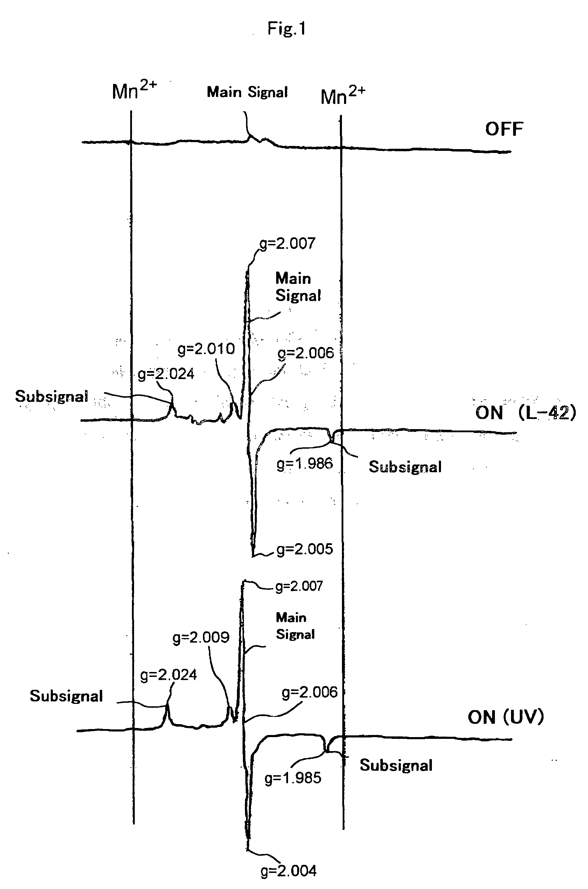

Fig. 1 shows conventional ESR spectra measured at 77 K in vacuum for the above-mentioned visible light responsive type material. In the figure, the upper segment is a spectrum measured in darkness and the middle segment is a spectrum measured with irradiation with light having a wavelength of not less than 420 nm (obtained by cutting off light of less than 420 nm in light from a mercury lamp). The lower segment is a spectrum obtained by irradiation with light from a mercury lamp from which light of less than 420 nm has not been cut off. The upper segment, middle segment, and lower segment are all results obtained at identical GAIN.

In the spectrum of the upper segment of Fig. 1, the main signal having a g value ranging from 2.004-2.007 was measured as being quite small and the two subsignals having g values of 1.985-1.986 and 2.024 were essentially not detected. Further, as is clear from a comparison of the upper segment and middle segment spectra in Fig. 1, in the middle segment spectrum, the main signal having a g value ranging from 2.004 to 2.007 and the two subsignals having g values ranging from 1.985 to 1.986 and 2.024 are substantially much greater in intensity than those of the upper segment spectrum. Further, as is clear from a comparison of the middle segment and lower segment spectra of Fig. 1, the intensity of the main signal having a g value of 2.004 to 2.007 and those of the two subsignals having g values of 1.985 to 1.986 and 2.024 essentially did not differ regardless of whether or not light of less than 420 nm was included in the irradiating light.

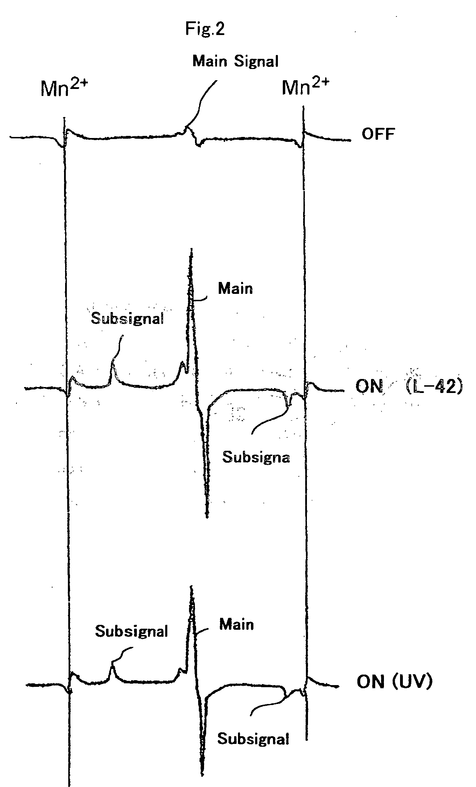

Further, as shown in Fig. 2, the main signal having a g value of 2.004-2.007 was observed to be quite small and the two subsignals having g values ranging from 1.985 to 1.986 and 2.024 were essentially not detected for the visible light responsive type material in darkness at ordinary temperature in vacuum. Further, it was found that the three signals were detected by ESR with irradiation by light having a wavelength of not less than 420 nm and light from a mercury lamp from which light of less than 420 nm had not been cut off at ordinary temperature in vacuum. In Fig. 2, the upper segment is a spectrum measured in darkness and the middle segment is a spectrum measured with irradiation by light having a wavelength of not less than 420 nm (obtained by cutting off light of less than 420 nm in light from a mercury lamp). The lower segment is a spectrum obtained by irradiation with light from a mercury lamp from which light of less than 420 nm was not cut off. The upper, middle, and lower segments are all the results of measurements made at identical GAIN.

In addition to the above-described signals, the visible light responsive type material may also have a subsignal having a g value ranging from 2.009 to 2.010 when measured with irradiation by light having a wavelength of not less than 420 nm at 77 K in vacuum. The subsignal having a g value ranging from 2.009 to 2.010 is shown in the ESR spectrum of the middle segment of Fig. 1.

As set forth above, the visible light responsive type material has a characteristic ESR signal. In addition, it is also a colored material. For example, when the reflectance for light with a wavelength of 600 nm is designated as 1 (or 100 percent), the reflectance for light with a wavelength of 450 nm can be not greater than 0.85 (or 85 percent), preferably not greater than 0.80 (or 80 percent), and still more preferably, not greater than 0.70 (or 70 percent). The greater the coloration, the greater the tendency toward visible light responsive activity. The reflectance referred to here is the result of measurement with a spectrophotometer. Although reflectance can also be measured with a color analyzer, the evaluation of the above-mentioned reflectance is conducted with a photospectrometer for better precision.

As set forth above, the visible light responsive type material has a characteristic ESR signal. In addition, it also has oxidation activity on NO for light in the visible range. Specifically, irradiation with visible light with a wavelength of at least 520 nm, or lower, results in NO oxidation activity. In the preferred material, irradiation with visible light with a wavelength of 570 nm or less results in NO oxidation activity.

The visible light responsive type material can be manufactured from a starting material in the form of amorphous or incomplete crystalline titanium oxide (including hydrous titanium oxide) and/or titanium hydroxide. The titanium compound starting material can be obtained by a wet method such as the sulfuric acid method or chloride method. More specifically, the titanium compound starting material can be obtained by hydrolyzing titanium chloride or titanium sulfate with ammonium hydroxide. Alternatively, the titanium compound starting material can be obtained by hydrolyzing titanium alkoxide with water or hydrolyzing titanium alkoxide with an ammonium hydroxide aqueous solution. However, from the perspective of the low cost of the starting materials, those obtained by hydrolysis of titanium chloride or titanium sulfate with ammonium hydroxide are preferred in industrial production. Accordingly, the hydrolysis of titanium chloride or titanium sulfate with ammonium hydroxide is described below.

The above-mentioned hydrolysis can be conducted, for example, by continuously or intermittently adding ammonium hydroxide aqueous solution to a titanium chloride aqueous solution or titanium sulfate aqueous solution, or continuously or intermittently adding a titanium chloride aqueous solution or titanium sulfate aqueous solution to an ammonium hydroxide aqueous solution. The concentrations of the titanium chloride aqueous solution, titanium sulfate aqueous solution, and ammonium hydroxide aqueous solution can be suitably determined. In the hydrolysis, the amount of ammonium hydroxide added is suitably adjusted to produce an alkaline reaction solution with a final pH of not less than 5. The titanium chloride may be in the form of titanium trichloride, titanium tetrachloride, or the like, or a mixture thereof. The hydrolysis can be conducted, for example, at a temperature of 0-100°C, preferably 20-80°C. However, hydrolysis at ordinary temperature is sometimes preferred from the viewpoint of obtaining titanium dioxide of comparatively low crystallinity or that is amorphous.

The hydrolysis product of titanium chloride or titanium sulfate with ammonium hydroxide is desirably washed with water or an ammonium hydroxide aqueous solution and employed as the starting material titanium compound. The washing of the hydrolysis product with water or ammonium hydroxide aqueous solution, for example, can be conducted by filtering the reaction solution comprising the hydrolysis product and further passing water or ammonium hydroxide aqueous solution through the hydrolysis product obtained as a filtrate. This method is preferred because of easy operation that it suffices to add water or ammonium hydroxide aqueous solution to the filtered hydrolysis product as it is and conduct nitration. In addition to the above, the washing of the hydrolysis product with water or ammonium hydroxide aqueous solution can be conducted by, for example, resuspending the hydrolysis product filtrate in water or ammonium hydroxide aqueous solution and filtering the suspension obtained. Washing with water or ammonium hydroxide aqueous solution is conducted to suitably decrease the quantity of residual ammonium salts such as ammonium chloride or ammonium sulfate produced during hydrolysis, and can be conducted multiple times.

Commercial products of amorphous or incomplete crystalline titanium dioxide may also be employed. Examples are incomplete crystalline titanium dioxide such as ST-01 and C-02 manufactured by Ishihara Sangyo.

In the manufacturing method, the starting material titanium compound such as amorphous or incomplete crystalline titanium oxide is heated in the presence of ammonia or a derivative thereof. The ammonia may be liquid or gaseous. When using ammonia gas, the starting material titanium compound is heated in an ammonia gas atmosphere. Examples of ammonia derivatives are ammonium salts such as ammonium hydroxide and ammonium chloride; the starting material titanium compound is heated, for example, in the presence of ammonium hydroxide or ammonium chloride.

The heating of the starting material titanium compound in the presence of ammonia or a derivative thereof is stopped when the absorbance of light at a wavelength of 450 nm by the material produced by the heating becomes greater than the absorbance of light at a wavelength of 450 nm by the startiug material titanium compound. Usually, the starting material titanium compound is white and its absorbance of light at a wavelength of 450 nm is about 10 percent. By contrast, when the starting material titanium compound is heated in the presence of ammonia or a derivative thereof, it gradually turns yellow. However, this coloration peaks at a certain point and then reduces, finally becoming a compound with absorbance about the same as the starting material titanium compound. Although the absorbance of light at a wavelength of 450 nm varies with the type of starting material titanium compound, type and quantity of ammonia (derivative) co-existed, heating temperature and time and the like, it sometimes reaches a maximum of about 60 percent. The characteristic of the visible light responsive type material is not definitively determined based on the intensity of absorbance of light at a wavelength of 450 nm, but when the absorbance of light at a wavelength of 450 nm is 15 percent or greater (reflectance is 85 percent or less), it has clearly become a material exhibiting visible light responsiveness. Accordingly, in the above-described heat treatment, it is desirable to set conditions so that when reflectance of light with a wavelength of 600 nm is defined as 1 (or 100 percent), the reflectance of light with a wavelength of 450 nm is 0.85 (or 85 percent) or less, preferably 0.80 (or 80 percent) or less, and more preferably, 0.70 (or 70 percent) or less. The absorbance referred to here is the result of measurement with a spectrophotometer.

The above-mentioned heating conditions cannot necessarily be specified by just temperature, but the temperature employed falls within a range of 250-550°C, for example. There is a tendency that the NOₓ elimination rate due to light with wavelengths of 420 and 470 nm is comparatively high with heating to within a range of 300-450°C, and even higher with heating to within a range of 325-425°C, and the NOₓ elimination rate due to light with wavelengths of 520 and 570 nm is comparatively high with heating to within a range of 325-450°C, and even higher with heating to within a range of 350-425°C. Accordingly, from the viewpoint of the high elimination rate of NOₓ in the visible light range, heating at a temperature ranging from 350-425°C is of greatest preference.

From the viewpoint of improving the NOₓ elimination rate for light within the visible range having wavelengths of 520 nm and 570 nm, a heating time of at least 30 min, preferably at least 1 hr, is desirable. Further, since no significant change in the NOₓ elimination rate was observed when the heating time was extended beyond 1 hr, the maximum heating time is about 3 hr.

The heating can be conducted at ordinary pressure. Further, the heating time can be suitably determined based on the absorbance of light with a wavelength of 450 nm by the material produced by heating.

The above-mentioned heating can be performed with one of the rotary kilns, tunnel kilns, muffle furnaces, or the like commonly employed in this field. When the individual grains of titanium oxide aggregate or sinter by heating, they can be comminuted as needed with a pulverizer.

Further, the material obtained by heating as set forth above can be washed with water or an aqueous solution as necessary. This washing sometimes improves the visible light responsiveness of the visible light responsive type material obtained. Further, based on conditions, a material having good visible light responsiveness can sometimes be obtained without washing.

When the amorphous or incomplete crystalline titanium oxide (the starting material titanium compound prior to heating) is those obtained, for example, by hydrolyzing titanium chloride with ammonium hydroxide, a considerable amount of ammonium chloride remains in the hydrolysis product. As a result, heating of the amorphous or incomplete crystalline titanium dioxide to a prescribed temperature as set forth above can convert it to a visible light responsive type material. However, a large quantity of ammonium chloride sometimes remains in the material obtained even after the heat treatment. In such cases, it is sometimes possible to remove the ammonium chloride by washing with water or a suitable aqueous solution to improve the visible light responsiveness of the visible light responsive type material.

In this case, washing the material obtained by heating with water or an aqueous solution is desirably conducted so that either the pH of the water or aqueous solution separated from the material following washing is 3.5 or greater (pH 3.5-7), or the quantity of chlorine ions (when titanium chloride is employed as the starting material of the hydrolysis product) or quantity of sulfuric acid ions (when titanium sulfate is employed as the starting material of the hydrolysis product) contained in the water or aqueous solution separated from the material following washing decreases.

The visible light responsive material is usually employed in the form of a powder. The granularity of the powder obtained can be adjusted by comminution or the like.

Silicon, aluminum, tin, zirconium, antimony, phosphorus, platinum, gold, silver, copper, iron, niobium, tungsten, tantalum, and other elements and compounds containing them can be coated on the surface of and/or carried within, or used to dope, the visible light responsive material as necessary. However, when used as an agent for teeth, the elements that are added are selected based on their effects on teeth.

The visible light responsive material mentioned above includes a photo responsive material which is a rutile titanium oxide containing nitrogen and will be activated by the action of light with a wavelength of greater than or equal to 470 nm. In particular, the photo responsive material is activated by the action of light with a wavelength of greater than or equal to 500 nm. In some cases, the photo responsive material is activated by the action of light from a light source with a peak wavelength of 630 nm and a half-width of 20 nm.

The activity of the above photo responsive material is estimated by measuring, for example, NOx oxidation activity (NO elimination activity). The measurement of NOx oxidation activity (NO elimination activity) can be done by using monochrome light with a half-width of 20 nm generated with an irradiation unit made by Nippon Bunko employing a 300 W xenon lamp as light source. The peaks wavelength of monochrome light can be, for example, 360nm, 420nm, 470nm, 520nm, 570nm, 630nm.

The photo responsive material exhibits, in some cases, NO₂ production activity even with the action of light with a peak wavelength of 570 nm and a half-width of 20 nm.

The visible light responsive photocatalyst used in the coating composition of the present invention may be a photo responsive material exhibits the NOx oxidation activity under the irradiation of visible light, specifically under the irradiation of light at least with a wavelength of from 400 to 600 nm. Conventional rutile titanium oxide exhibits some extent of activity to visible light around 400nm. For example, a reference catalyst of the Japan Catalyst Association JRC-TI03 (rutile) shows weak activity to the light ranging up to 470nm as shown in Table 5 but does not have any activity to the light greater than 500nm. Material exhibiting photocatalytic activity to visible light with a wavelength of greater than or equal to 470 nm, especially higher than 500nm to around 600nm has not been known.

With the above-mentioned reference catalyst of the Japan Catalyst Association JRC-TI03 (rutile), NOx oxidation activity (NO elimination activity) obtained in irradiation of a light with a wavelength of 470 nm is about 10% on the basis of NOx oxidation activity (NO elimination activity) obtained in irradiation of a wavelength of 360 nm. With irradiation of a wavelength of 520 nm, it exhibits no activity. In contrast, the above-mentioned photo responsive material exhibits NOx oxidation activity (NO elimination activity) with irradiation of a monochrome light with a wavelength of 360nm to 570 nm at least and in some cases, it exhibits NOx oxidation activity (NO elimination activity) with irradiation of a monochrome light with a wavelength of 630nm.

Specifically, NOx oxidation activity (NO elimination activity) obtained in irradiation of a light with a wavelength of 470 nm is about 80%, that obtained with a light of a wavelength of 520 nm is about 50%, and that obtained with a light of a wavelength of 570 nm is about 10%.

The above-mentioned photo responsive material is rutile titanium oxide containing nitrogen. Presence of nitrogen is confirmed by measurement of Ti-N bonding in X-ray photoelectron spectroscopy. Content of nitrogen is calculated from area of peak assigned to 1s electron of nitrogen bonding to titanium measured in the X-ray photoelectron spectroscopy. Content of nitrogen can suitably be adjusted such a level that the photo responsive material has visible light responsive properties.

For example, the area (N1s) can be in the range of 0.01 to 10 or in the range of 0.1 to 5 on the basis that the area of (Ti2p3/2) and (Ti2p1/2) is set to 100.

It is confirmed that the above-mentioned photo responsive material is rutile titanium oxide from the fact that the raw material titanium oxide is rutile type and the measurement of the photo responsive material with XDR.

The photo responsive material has the ratio of oxygen and nitrogen against titanium being nearly the stoichiometric ratio. The fact that the ratio of oxygen and nitrogen against titanium is nearly the stoichiometric ratio can be identified, for example, by the ratio ((O1s+N1s)/Ti2p) of the areas of peak assigned to 1s electron of oxygen bonding to titanium and peak assigned to 1s electron of nitrogen bonding to titanium against the area of peak assigned to 2p electron of titanium measured in the X-ray photoelectron spectroscopy. The photo responsive material used in the present invention has the ratio (O1s+N1s)/Ti2p being 1.95 or more and 2.05 or less. More preferably, the ratio (O1s+N1s)/Ti2p ranges from 1.97 to 2.03.

The above photo responsive material can be prepared by a method comprising treatment of rutile titanium oxide with nitrogen plasma. The rutile titanium oxide used in this method may be either a rutile titanium oxide prepared by a wet method or a rutile titanium oxide prepared by a dry method. The material can be obtained by a method treating rutile titanium oxide with nitrogen plasma, preferably the treatment is carried out in the state that invasion of atmosphere is substantially precluded.

Nitrogen plasma treatment is carried out by generating nitrogen plasma by introducing nitrogen gas to rutile titanium oxide irradiated with an electromagnetic wave, such as a microwave or a radio wave under redued pressure and by exposing the rutile titanium oxide to the plasma for a prescribed period of time. The reduced pressure may be 10 Torr or less, or 2 Torr or less. The power of the electromagnetic wave can be determined appropriately in view of the amount of rutile titanium oxide to be treated and the generating state of plasma. The quantity of nitrogen gas to be introduced can be determined appropriately in view of the state of reduced pressure and the generating state of plasma. The time period of exposure of retile titanium oxide to the nitrogen plasma can be determined appropriately in view of the amount of nitrogen to be introduced into the rutile titanium oxide and photo responsiveness, especially, to visible light.

The preparation method is preferably carried out in the state that invasion of atmosphere into a plasma container is substantially precluded, and the state that invasion of atmosphere into a plasma container is substantially precluded means the state that it requires 10 minutes for variation of vacuum level in a sealed space by 1 Torr. It is inferred that less invasion of atmosphere may lead easy introduction of nitrogen into rutile titanium oxide. The nitrogen plasma may optionally contain a gas other than nitrogen.

This preparation method of material can be applied not only to a power but also to rutile titanium oxide fixed on a substrate with or without an appropriate binder.

The photo responsive material may be prepared by a method comprising ion implantation nitrogen into rutile titanium oxide. The rutile titanium oxide used in this method may be either a rutile titanium oxide prepared by a wet method or a rutile titanium oxide prepared by a dry method. The ion implantation can be carried out using a method and an apparatus used in the semiconductor industry. Conditions of ion implantation can be determined in view of the amount of nitrogen to be introduced into the rutile titanium oxide and the state of the rutile titanium oxide.

### [Dental Fluorine Coating Method]

The dental fluorine coating method of the present invention comprises the steps of applying the fluorine coating composition of the present invention to the surface of a tooth and irradiating the coating with light. The fluorine coating composition may be applied to the surface of the tooth with a blade, brush, or the like; by affixing a product obtained by coating or impregnating a substrate (desirably of a light-passing substance) with the fluorine coating composition in advance; or the like.

Since a substantive effect is achieved only at the contact surface with the tooth and the effect is not improved with the amount applied, it is adequate that the fluorine coating composition is applied to the surface of the tooth in a thickness that does not impede the passage of light.

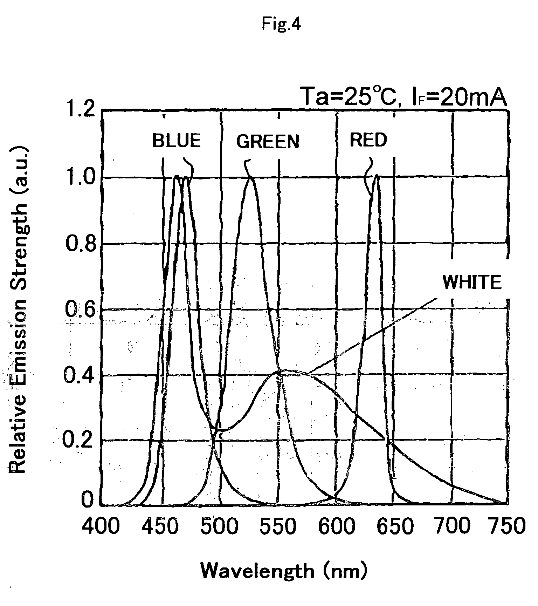

The light used for irradiation can be suitably selected based on the type of photocatalyst contained in the fluorine coating composition When the photocatalyst is visible light responsive titanium oxide, the irradiating light comprises at least one wavelength component of greater than or equal to 420 nm, preferably a wavelength component of from 420 to 600 nm. For example, the irradiating light may comprise at least a wavelength component of from 450 to 600 nm. The light, particularly light comprising a wavelength component of greater than or equal to 420 nm, may be generated by a photopolymerizer, light-emitting diode, or halogen lamp, or be plasma light, for example. The photopolymerizer may be one that is employed in visible light polymerization to cure dental resins. The light-emitting diode either has a light-emission wavelength in the visible light range of greater than or equal to 420 nm, or has a light-emission wavelength exclusively in the visible light range. Examples of such light-emitting diodes are violet-emitting diodes, blue-emitting diodes, green-emitting diodes, yellow-emitting diodes, and white-emitting diodes. Violet-emitting diodes have a light emission wavelength from the ultraviolet range to the visible light range. Blue-emitting diodes, green-emitting diodes, yellow-emitting diodes, and white-emitting diodes have light-emitting wavelengths exclusively within the visible light range. The light-emission spectra of a blue-emitting diode (BLUE), green-emitting diode (GREEN), and white-emitting diode (WHITE) are shown in Fig. 4.

The device described in Japanese Unexamined Patent Publication (KOKAI) 2000-217844 may be employed as a dental irradiation device with a built-in light-emitting diode. This dental irradiation device employs multiple light-emitting diodes positioned in a curved manner in front of a row of teeth to uniformly irradiate and remove coloration from an entire row of teeth.

The dental fluorine coating method of the present invention has a fluorine coating effect on enamel, cementum; and dentin. Not only is the dentin coated with fluorine, but the tooth surface is hardened. Secondarily, a smarting sensation (hypersensitivity) in the teeth can sometimes be reduced or eliminated.

### Embodiments

Embodiments of the present invention are given below. However, the present invention is not limited thereto.

### Embodiment 1

### (Dental Fluorine Coating)

A 0.5 g quantity of 2 percent sodium fluoride - phosphoric acid solution (Fluoragel; import source: Hakusui Trading Company) and 20 mg of the visible light responsive titanium oxide prepared in Manufacturing Example 3 were added, intimately kneaded, and then uniformly coated to about 0.5 mm on the tooth (denoted as tooth alpha) of Subject A. Following the application, the surface of tooth alpha was irradiated for one minute with a commercial photopolymerization device (D-LUX10 made by Dentrade Co.).

As a result, upon visual inspection, the irradiated surface had a more natural luster than prior to treatment. Further, the hardness increased by a pencil hardness of 2 to 3. When Subject A had previously filled his mouth with water, tooth alpha had smarted, but the sensation disappeared.

### Manufacturing Example 1

To pure ice water (two liters of water) were added 500 g of titanium tetrachloride (special grade, manufactured by Kanto Kagaku K.K.) and the mixture was stirred and dissolved, yielding a titanium tetrachloride aqueous solution. While stirring 200 g of this aqueous solution in a stirrer, about 50 mL of ammonia water (comprising 13 weight percent as NH₃) was added as rapidly as possible. The quantity of ammonia water added was adjusted to yield a final pH of about 8 in the aqueous solution. This converted the aqueous solution to a white slurry. After stirring for another 15 min, the mixture was filtered with a suction filter. The filtered precipitate was dispersed in 20 mL of ammonia water (containing 6 weight percent as NH₃) and stirred using a stirrer for about 20 hours. The mixture was then suction filtered again, yielding a white hydrolysis product.

The white hydrolysis product obtained was transferred to a crucible and heated to 400°C for 1 hour in air with an electric furnace, yielding a yellow product.

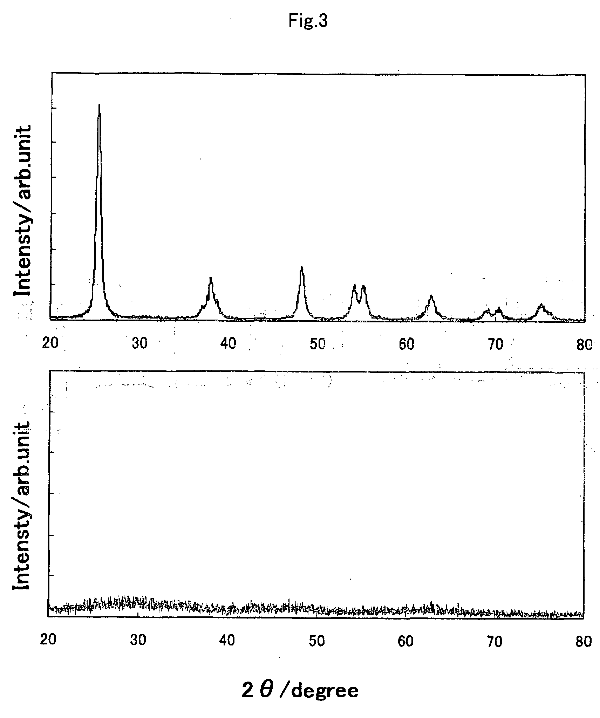

The results of XRD measurement of the obtained product are given in the upper segment of Fig. 3. In addition, the results of XRD measurement of the white hydrolysis product dried at 50°C are given in the lower segment of Fig. 3. From these results, it will be understood that the product obtained by drying the white hydrolysis product at 50°C was amorphous and the obtained product contained anatase titanium dioxide.

The diffuse reflectance spectra of the obtained product and the white hydrolysis product dried at 50°C obtained were measured under the following conditions with a Hitachi Autorecording Spectrophotometer (U-3210) equipped with integrating sphere:

| | |
|---|---|
| Scan speed | 120 nm/min, |
| Response | MEDIUM, |
| Band pass | 2.00 nm, and |
| Reference | barium sulfate. |

As a result, when the reflectance at 700 nm of the obtained product was defined as 100 percent, the reflectance thereof at 450 nm was 61 percent. By the contrast, when the reflectance at 700 nm of the product obtained by drying the white hydrolysis product at 50°C was denned as 100 percent, the reflectance thereof at 450 percent was 95 percent.

Further, the ESR spectrum of the obtained product was measured. Measurement was conducted at 77 K or ordinary temperature in vacuum (0.1 Torr or less). The measurement conditions were as follows:

### [Basic Parameters]

| | |
|---|---|
| Measurement temperature | 77 K or ordinary temperature |
| Field | 324 mT ± 25 mT |
| Scan time | 4 min |
| Mod. | 0.1 mT |
| Receiver GAIN | 10-100 (measurement sensitivity) |
| Time constant | 0.1 sec |
| Light source | 500 W high-pressure mercury lamp |
| Filter | L-42 |

### [Sample Preparation]

| | |
|---|---|
| Vacuum evacuation | 1 hr or more |

### [g Value Calculation]

g=gₘₙ x Hₘₙ/(Hₘₙ + ΔH) based on Mn²⁺ marker (gₘₙ=1.981 (third from the high magnetic field side)
Hₘₙ: Mn²⁺ marker magnetic field
ΔH: Amount of change in magnetic field from Hₘₙ

Fig. 1 (measurement temperature 77 K) and Fig. 2 (measurement temperature ordinary temperature) show the ESR spectra in the dark in the upper segment, the ESR spectrum measured with irradiation with light through a filter (L-42) cutting light (obtained using a 500 W high-pressure mercury lamp) below 420 nm in the middle segment, and the ESR spectrum measured with irradiation of light employing a 500 W high-pressure mercury lamp without using a filter (L-42) cutting light below 420 nm is shown in the lower segment.

A comparison of the spectra of the upper and middle segments of Fig. 1 clearly reveals that in the middle segment spectrum, the main signal having a g value ranging from 2.004 to 2.007 and the two subsignals having g values ranging from 1.985 to 1.986 and 2.024 exhibited intensities stronger than in the spectrum in the upper segment. Further, a comparison of the spectra of the middle and lower segment spectra of Fig. 1 clearly reveals that neither the main signal having a g value ranging from 2.004 to 2.007 nor the two subsignals having g values ranging from 1.985 to 1.986 and 2.024 differed substantially even when light of less than 420 nm was included in the irradiating light.

Further, as shown in Fig. 2, the above-described three signals were observed in the ESR of the visible light responsive type material of Manufacturing Example 1 in darkness and with irradiation with light having a wavelength of 420 nm or more at ordinary temperature in air.

The main signal having a g value ranging from 2.004 to 2.007 and the two subsignals having g values ranging from 1.985 to 1.986 and 2.024 were not observed under any of the ESR measurement conditions in the white hydrolysis product dried at 50°C.

### Manufacturing Example 2

A 3 g quantity of the powder obtained in Manufacturing Example 1 was suspended in 100 mL of pure water and stirred for 1 hr with a magnetic stirrer. The solution obtained was suction filtered. Sample remaining on the filter paper was again stirred in pure water and suction filtered. Filtration was repeated three times until the filtrate reached 6-7 on pH test paper.

The powder obtained was left standing for a day in a drier set to 110°C and dried, yielding the visible light responsive type material.

### Manufacturing Example 3

A 23 kg quantity of titanium tetrachloride was gradually added to 207 kg of water having a temperature of 0°C held in a 300 L reaction vessel (cooling and stirring are available). At that time, the temperature of the aqueous solution reached a maximum of 6°C. The titanium chloride was stirred for two days to prepare a transparent titanium tetrachloride aqueous solution. When 12.5 percent ammonia water was added dropwise while stirring the titanium tetrachloride aqueous solution that had been prepared, the solution gradually clouded over. The amount of ammonia water was adjusted so that the clouded solution reached pH 8.

The clouded solution was suction filtered. The white precipitate remaining on the filter paper weighed 131 kg. The white precipitate was dispersed in 200 kg of ammonia water (6 percent as NH₃), stirred for 24 hr, and suction filtered. The white precipitate following filtering weighed 108 kg. The white precipitate was placed in a forced ventilation shelf-type drier set to 50°C and dried for four days. Following drying, the sample weighed 17 kg.

A 1 kg quantity of the dried sample was placed in an alumina crucible (20×20×5 cm), the crucible was placed in a gas furnace, a thermocouple was placed on the surface of the sample, and the sample was sintered for 1 hr so that the sample temperature reached 400°C.

A 3 g quantity of the powder obtained was suspended in 100 mL of pure water and stirred for 1 hr with a magnetic stirrer. The solution obtained was suction filtered. Sample remaining on the filter paper was again stirred in pure water and suction filtered. Filtration was repeated three times until the filtrate reached 6-7 on pH test paper.

The powder obtained was left standing for a day in a drier set to 110°C and dried, yielding the visible light responsive type material.

### Manufacturing Example 4 (Method of manufacturing visible light responsive material from titanium sulfate (1))

Titanium sulfate (IV) aqueous solution (product name: Titanium sulfate (IV), made by Kanto Kagaku K.K. (Shika first grade, an aqueous solution comprising not less than 24 weight percent of titanium sulfate (IV)) was employed without alteration as titanium sulfate (IV) solution. While mixing 50 g of this aqueous solution with a stirrer, 58 mL of ammonia water (ammonia solution as provided:water = 1:1) was added as rapidly as possible with a buret. When stirring was continued, clouding began and gradually increased viscosity. Ammonia water was further added to adjust the pH to 7 as indicated by universal test paper. When 24 hr had elapsed, filtration was conducted with a suction filter. The white product on the filter paper was stirred in ammonia water that had been adjusted to pH 11, filtering was repeated 8 times, and washing was conducted, yielding a white powder. The powder obtained was dried at 50°C, yielding sample powder. The BET surface area of the hydrolysis product obtained (sample powder) was 308.7 m²/g. An 8 g quantity of the obtained sample powder was charged to a crucible, transferred to an electric furnace, and sintered for 60 min at 400°C, yielding 6.3 g of a clear yellow powder with a BET surface area of 89.4 m²/g.

The resulting sample powder was subjected to X-ray diffraction test (XRD) to reveal that it comprises anatase titanium oxide. X-ray photoelectron spectroscopy (XPS) of the resulting sample powder was measured with an X-ray photoelectron spectral analyzer unit (product name: Quantum 2000, made by Ulvacphi (K.K.)). As a result, the abundance ratio (O/Ti) of elemental oxygen and elemental titanium calculated from the area of the peak attributed to the 2p electron of titanium and the area of the peak attributed to the 1s electron of oxygen obtained shows that the powder has oxygen defects in the crystal structure. Due to this oxygen defects, it is believed that the powder shows clear or slight yellow color and visible light activity (photocatalytic activity).

The ESR spectrum of the powder obtained was measured. Measurement was conducted at 77 K in vacuum (0.1 Torr). The measurement conditions were identical to those in Manufacturing Example 1.

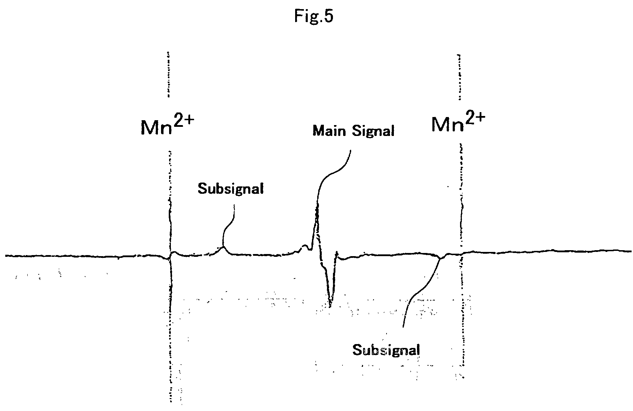

Fig. 5 shows the ESR spectra measured with irradiation with light through a filter (L-42) cutting out light (obtained using a 500 W high-pressure mercury lamp) below 420 nm (measurement temperature of 77 K). Although the ESR spectrum in darkness was also measured, essentially no signal was observed.

In the spectrum shown in Fig. 5, a main signal having a g value ranging from 2.004 to 2.007 and two subsignals having g values ranging from 1.985 to 1.986 and 2.024 were observed.

Neither a main signal having a g value ranging from 2.004 to 2.007 nor the two subsignals having g values ranging from 1.985 to 1.986 and from 1.985 to 2.024 were observed in product obtained by drying white hydrolysis product at 50°C under any of the ESR measurement conditions.

### Manufacturing Example 5 (Method of Manufacturing Visible Light Responsive Material from titanium sulfate (2))

A 50 g quantity of 24 percent titanium sulfate solution (Shika first grade, made by Kanto Kagaku) was added to 400 mL of distilled water and stirred with a magnetic stirrer. Concentrated ammonia water (28 percent, Kanto Kagaku, special grade) was added thereto and a neutralization reaction was conducted. Following the neutralization reaction, the mixture was adjusted to pH 7 and stirred for 15 min. Since the stirrer was sometimes no longer able to turn at that time, distilled water was added (200 mL). Fifteen minutes later, after stirring had been stopped, the mixture was left standing for some time and the supernatant was discarded. Filtration was conducted with a nutsche filter and the filtrate was washed with 2 L of ammonia water (5:95). This operation is a method wherein ammonia water was added when the mixture formed cakes on the filter paper. The mixture obtained was then dried for 24 hr at 60°C and sintered for 1 hr at 400°C, yielding the visible light responsive type material.

A 0.2 g quantity of the sample prepared in the above was coated on a glass plate (6x6cm) and the plate was placed in a Pyrex glass reaction vessel (internal diameter 160 mm, thickness 25 mm). Monochrome light with a half-width of 20 nm was irradiated with an irradiation unit made by Nippon Bunko employing a 300 W xenon lamp as light source. A 1.5 L/min flow of 0 percent RH in humidity simulated polluted air (NO: 1 ppm) was continuously fed into the reaction vessel and the change in concentrations of NO and NO₂ were monitored at the reaction discharge outlet. The NO concentration was measured by the chemoluminescence method using ozone. The NOₓ elimination rate (=NO elimination rate - NO₂ generation rate) was obtained from the cumulative value of the values monitored over 1 hr for each measurement wavelength. Nitrogen oxides analyzer Model 8840 available from Monitor labs Inc was used for measurement of NO concentration. The measurement results are given in Table 1.

**Table 1**

| Wavelength (nm) | NO reduction (%) | NO₂ generation (%) | NOx elimination (%) |
|---|---|---|---|
| 570 | 1.2 | 0 | 1.2 |
| 520 | 9.8 | 1.2 | 8.6 |
| 470 | 21.5 | 4.3 | 17.2 |
| 420 | 22.2 | 5.5 | 16.6 |
| 360 | 28.5 | 10.3 | 18.2 |

The ESR spectrum of the material obtained was measured. The measurement was conducted in vacuum (0.1 Torr) at 77 K. The measurement conditions were identical to those in Manufacturing Example 1.

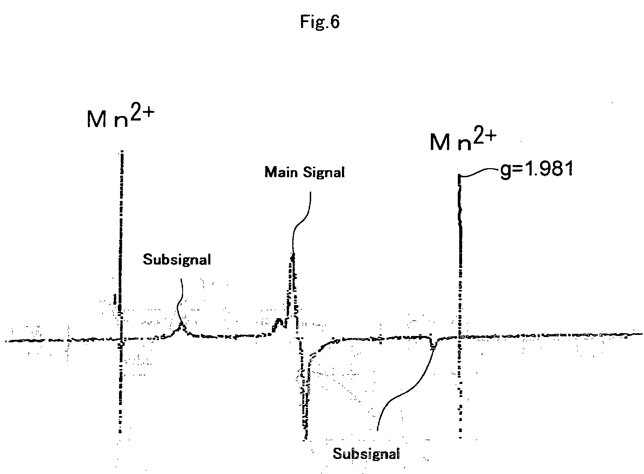

Fig. 6 (measurement temperature of 77 K) gives the ESR spectrum measured with irradiation by light passing through a filter (L-42) cutting out light (obtained using a 500 W high-pressure mercury lamp) below 420 nm.

A main signal having a g value ranging from 2.004-2.007 and two subsignals having g values ranging from 1.985 to 1.986 and 2.024 were observed in the spectrum of Fig. 6.

Neither a main signal having a g value ranging from 2.004-2.007 nor two subsignals having g values ranging from 1.985 to 1.986 and 2.024 were observed under any of the ESR measurement conditions in the product obtained by drying the white hydrolysis product at 50°C.

### Manufacturing Example 6 (Method of Manufacturing from Alkoxide)

To 200 g of pure water, 30 g of titanium isopropoxide were gradually added with stirring (with a molar ratio of water to titanium isopropoxide of about 10:1). The solution obtained was stirred for about 30 min and the precipitate (hydrolysis product) was recovered by filtration. The precipitate (hydrolysis product) was then suspended in pure water, stirred for one day, filtered, dried at 110°C, and sintered for 1 hr at 400°C. The white powder obtained was referred to as sample A.

With the exception that the precipitate (hydrolysis product) was suspended in ammonia water (ammonia concentration: 6 percent) and stirred for one day instead of being suspended in pure water and stirred for one day, a yellow powder referred to as sample B was obtained by the same operation as above.

The NO activity of samples A and B were measured by the above-described test method. The results are given in Table 2 below.

**Table 2**

| NO Activity | | | |
|---|---|---|---|
| Sample A | | | |
| Wavelength (nm) | NO elimination (%) | NO₂ generation (%) | NOx elimination (%) |
| 570 | 0 | 0 | 0 |
| 520 | 0.7 | 0 | 0.7 |
| 470 | 1.4 | 0 | 1.4 |
| 420 | 1.9 | 0 | 1.9 |
| 360 | 16.0 | 2.5 | 13.5 |

| Sample B | | | |
|---|---|---|---|
| Wavelength (nm) | NO elimination (%) | NO₂ generation (%) | NOx elimination (%) |
| 570 | 1.0 | 0 | 1.0 |
| 520 | 4.7 | 0 | 4.7 |
| 470 | 15.6 | 3.4 | 12.2 |
| 420 | 16.4 | 4.6 | 11.8 |
| 360 | 19.1 | 10.0 | 9.1 |

From the results of Table 2, it will be understood that a material comprised of titanium oxide having visible light responsiveness was obtained by heat treating titanium oxide (titanium hydrolysis product) in the presence of ammonia.

The ESR spectra of the materials obtained were measured. The measurement was conducted at 77 K in vacuum (0.1 Torr). The measurement conditions were identical to those in Manufacturing Example 1.

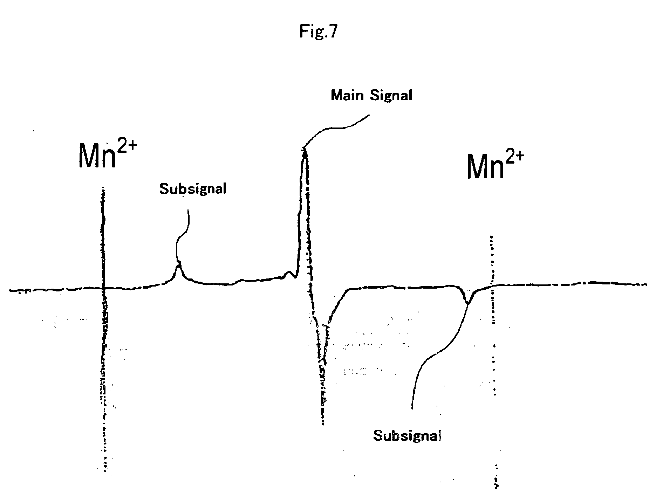

Fig. 7 (measurement temperature 77 K) gives the ESR spectra measured with irradiation by light passing through a filter (L-42) cutting out light (obtained using a 500 W high-pressure mercury lamp) below 420 nm.

A main signal having a g value ranging from 2.004-2.007 and two subsignals having g values ranging from 1.985 to 1.986 and from 1.985 to 2.024 were observed in the spectra of Fig. 7.

Neither a main signal having a g value ranging from 2.004-2.007 nor two subsignals having g values ranging from 1.985 to. 1.986 and 2.024 were observed under any of the ESR measurement conditions in the product obtained by drying the white hydrolysis product at 50°C.

### Manufacturing Example 7

A 10 g quantity of Catalyst Society Reference Catalyst JRC-TIO-3 (rutile) was charged to a 400 mL silica test tube. The silica test tube was connected to a plasma generating device, the interior of the system was evacuated with a vacuum pump, and N₂ gas was introduced (at a flow rate of 100 sccm) into the system to achieve a pressure of about 1 Torr. The titanium dioxide in the test tube was irradiated with 1,200 W electromagnetic waves (2.45 GHz) to generate a plasma. The rutile titanium dioxide powder in the test tube was processed for five minutes while stirring.

Without introducing gas into the plasma processing system, and with pump evacuation halted, it took about 40 minutes for the degree of vacuum to rise 1 Torr.

XRD of the sample obtained revealed that the powder contained rutile titanium oxide. The results of XPS measurement of the sample obtained are given below. An X-ray photoelectron analyzer (ESCA Model 750 made by Shimadzu Seisakujo (K.K.)) was employed in XPS measurement.

X-ray photoelectron spectroscopy (XPS) of the rutile titanium oxide powder obtained was used to compute the areas of the peaks belonging to the titanium 2p electrons (458.9 eV (Ti 2p 3/2)) and (464.3 eV (Ti 2p 1/2)), the area of the peak belonging to the 1s electron of oxygen bonded to the titanium (530.0 eV (O 1s)), and the area of the 1s electron of the nitrogen (393.5 eV (N 1s)). The area ratio obtained ((O 1s + N 1s)/Ti 2p) was 1.98. The areas of (458.9 eV (Ti 2p 3/2)) and (464.3 eV (Ti 2p 1/2)) were 12,411 cps • eV. The area of 530.0 eV (O 1s) was 24,462 cps • eV And the area of 393.5 eV (N 1s) was 273 cps • eV.

X-ray photoelectron spectroscopy (XPS) measurement was also conducted on rutile titanium oxide powder (Catalyst Society Reference Catalyst JRC-TIO-3 (rutile)) that had not been plasma treated, revealing no peak belonging to the nitrogen 1s electron. The area ratio (O 1s/Ti 2p) of the peaks belonging to the 2p electrons of titanium (458.9 eV (Ti 2p 3/2) and 464.5 eV (Ti 2p 1/2)) and the peak belonging to the 1s electron of the oxygen bonded to the titanium (529.8 eV (O 1s) was 1.99.

### Manufacturing Example 8

A 10 g quantity of Catalyst Society Reference Catalyst JRC-TIO-3 (rutile) was charged to a 400 mL silica test tube. The silica test tube was connected to a plasma generating device, the interior of the system was evacuated with a vacuum pump, and N₂ gas was introduced (at a flow rate of 100 sccm) into the system to achieve a pressure of about 1 Torr. The titanium dioxide in the test tube was irradiated with 600 W electromagnetic waves (2.45 GHz) to generate a plasma. The rutile titanium dioxide powder in the test tube was processed for 10 minutes while stirring, and left as it was for 10 minute, and then processed for 20minutes.

Without introducing gas into the plasma processing system, and with pump evacuation halted, it took about 40 minutes for the degree of vacuum to rise 1 Torr.

XRD of the sample obtained revealed that the powder contained rutile titanium oxide. The results of XPS measurement of the sample obtained are given below.

X-ray photoelectron spectroscopy (XPS) of the rutile titanium oxide powder obtained was used to compute the areas of the peaks belonging to the titanium 2p electrons (459.2 eV (Ti 2p 3/2)) and (464.7 eV (Ti 2p 1/2)), the area of the peak belonging to the 1s electron of oxygen bonded to the titanium (530.1 eV (O 1s)), and the area of the 1s electron of the nitrogen (394.8 eV (N 1s)). The area ratio obtained ((O 1s + N 1s)/Ti 2p) was 1.97. The areas of (459.2 eV (Ti 2p 3/2)) and (464.7 eV (Ti 2p 1/2)) were 14,565 cps • eV. The area of 530.1 eV (O 1s) was 28,430 cps • eV. And the area of 394.8 eV (N 1s) was 102 cps • eV.

The area ratio (O 1s/Ti 2p) of rutile titanium oxide powder that had not been plasma treated was 1.99.

### Industrial Applicability

The present invention provides a fluorine coating agent employing a photocatalyst and a fluorine coating method that provide a desired fluorine coating effect more rapidly than prior art.

## Claims

1. A composition for dental fluorine coating comprising a fluorine-containing compound and a photocatalyst.

2. The composition of claims 1, wherein the photocatalyst is UV-responsive titanium oxide or visible light responsive titanium oxide.

3. The composition of claim 2, wherein the visible light responsive titanium oxide is a photocatalyst (hereinafter referred to as a "visible light responsive photocatalyst") that is activated by the action of light with a wavelength of greater than or equal to 420 nm.

4. The composition of claim 3, wherein the visible light responsive photocatalyst is activated by the action of light with a wavelength of greater than or equal to 450 nm.

5. The composition of claim 3 or 4, wherein the visible light responsive photocatalyst is a visible light responsive type material comprising at least anatase type titanium dioxide, as well as the main signal having a g value ranging from 2.004 to 2.007 and two subsignals having a g value ranging from 1.985 to 1.986 and a g value at 2.024 are observed in the ESR measured under the irradiation of light having a wavelength of 420 nm or more at 77 K in vacuum, and said three signals are observed in a little amount or substantially not observed in darkness at 77 K in vacuum.

6. A dental fluorine coating method comprising the steps of applying the composition of any of claims 1 to 5 to the surface of a tooth and irradiating the resultant coating with light.

7. A dental fluorine coating method comprising the steps of applying the composition of any of claims 3 to 5 to the surface of a tooth and irradiating the resultant coating with light comprising a component with a wavelength of greater than or equal to 420 nm.

8. The dental fluorine coating method of claim 6 or 7, wherein the light is emitted by a photopolymerizer, light-emitting diode, or halogen lamp, or is plasma light.

9. The dental fluorine coating method of claim 8, wherein the light-emitting diode is a violet-emitting diode, blue-emitting diode, green-emitting diode, yellow-emitting diode, or white-emitting diode.

10. The dental fluorine coating method of any of claims 6 to 9, wherein a smarting sensation (hypersensitivity) in the teeth is reduced or eliminated.
